# FASCICULE DE BREVET EUROPEEN

(11) **EP 3 166 543 B1**
(45) Date de publication et mention de la délivrance du brevet: **09.05.2018**
(21) Numéro de dépôt: 15753091.6
(22) Date de dépôt: 02.07.2015
(51) Int. Cl.: A61F 2/42, A61B 17/16, A61F 2/30

(54) **ENSEMBLE POUR L'ARTHROPLASTIE DU PIED**
ANORDNUNG FÜR FUSSARTHROPLASTIE
ASSEMBY FOR FOOT ARTHROPLASTY

(30) Priorité: 10.07.2014 FR 1456673
(43) Date de publication de la demande: 17.05.2017
(73) Titulaire: TORNIER, 38330 Montbonnot-Saint-Martin (FR)
(72) Inventeur: COLOMBIER, Jean-Alain, F-31130 Balma (FR)
(74) Mandataire: Lavoix
(86) Numéro de dépôt international: PCT/FR2015/051832
(87) Numéro de publication internationale: WO 2016/005684

(56) Documents cités:
- EP-A1- 1 508 316
- WO-A1-2013/177252
- DE-U1- 29 514 169
- GB-A- 1 320 956

## Description

L'invention concerne un lot d'implants pour effectuer une arthroplastie du pied, c'est-à-dire pour assurer, de manière chirurgicale, la réfection d'une articulation du pied, qu'elle soit interphalangienne ou métatarsophalangienne.

On connaît diverses techniques pour assurer la réfection d'une articulation. Ainsi le document WO-2010/079288 divulgue un implant orthopédique utilisé en arthroplastie digitale comprenant un premier élément destiné à être implanté dans une phalange proximale et un second élément destiné à être implanté dans une phalange adjacente distale, chaque élément comportant une tige d'implantation dans l'os et une tête d'articulation interphalangienne, la tête du premier élément présentant une surface biconvexe d'articulation avec une vallée centrale et la tête du second élément présentant une surface biconcave d'articulation agencée pour coopérer avec la dite surface biconvexe et comportant une crête centrale.

Dans le cas d'une arthroplastie du pied, il est connu d'utiliser un élément formé de deux tiges s'étendant l'une à l'opposé de l'autre à partir d'une partie médiane en forme de boule. Les tiges sont de longueurs différentes, la tige destinée à être implantée dans une phalange dite distale -dite tige distale - étant plus courte que la tige destinée à être implantée dans une phalange dite proximale - dite tige proximale (il est rappelé que la phalange distale est plus éloignée de la cheville que la phalange proximale et est donc plus courte que cette dernière). Le matériau constitutif d'un tel implant monobloc est choisi en sorte d'avoir une flexibilité compatible avec celle recherchée pour l'articulation à reconstituer (en pratique du silicone).

Lors de la pose d'un tel implant, le chirurgien commence par inciser le tendon commandant le mouvement de la phalange distale par rapport à la phalange proximale et un foret est utilisé pour forer un canal dans l'une puis l'autre des phalanges avant d'y insérer la tige correspondante.

En pratique un choix doit être fait entre plusieurs tailles d'implants en fonction de la configuration de l'articulation à refaire ; on comprend en effet que, plus l'articulation à reconstituer est grande, plus l'implant doit être gros ; il y a couramment entre 2 et 5 tailles différentes au sein d'un lot d'implants. A titre d'exemple, si l'on considère deux implants de tailles successives, on peut avoir des tiges de mêmes diamètres et de mêmes longueurs, mais des parties médianes de diamètres différents, ou au contraire des parties médianes de mêmes dimensions séparant des tiges de diamètres et de longueurs différentes. En fonction du diamètre de l'implant choisi, le chirurgien choisit un foret de diamètre approprié et fore un canal de longueur à peine supérieure à celle de la tige correspondante.

Ainsi, un lot d'implants comporte classiquement une pluralité d'implants ainsi qu'une pluralité de forets ; un exemple est fourni par EP 1 508316. Par ailleurs, les implants étant habituellement obtenus par moulage, il faut autant de moules bien différents qu'il y a d'implants dans le lot considéré.

L'invention vise à simplifier la constitution d'un lot de tels implants pour une arthroplastie du pied, voire sa fabrication.

Elle propose à cet effet un lot d'implants pour une arthroplastie du pied comportant une pluralité d'implants de tailles croissantes et d'un foret unique, chaque implant comportant à partir d'une portion médiane une tige proximale et une tige distale plus courte que la tige proximale, ces tiges et ce foret présentant des conicités égales comprises entre 2 et 6° ; de préférence, les tiges et le foret ont une conicité de 4.8° environ.

De manière préférée, la tige distale d'un implant a la même longueur que la tige proximale d'un autre implant, ce qui simplifie la formation des moules de fabrication des implants d'un même lot.

De manière avantageuse, le foret comporte sur sa tige de travail au moins un repère visuel définissant, à partir de la pointe de ce foret, une distance de pénétration égale à la longueur d'au moins une tige distale ou proximale d'un implant dudit lot. Cela permet au chirurgien de bien estimer la profondeur à laquelle il doit forer un canal destiné à recevoir une tige donnée d'un implant choisi.

De manière préférée, les implants, classés par ordre de taille croissante, ont chacun (sauf le plus grand) une tige proximale dont la longueur est égale à celle de la tige distale de l'implant suivant.

De manière avantageuse, le foret comporte sur sa tige de travail, pour chacune des longueurs différentes des tiges des implants dudit lot, un repère visuel. Toutefois il suffit d'un nombre de repères égal à N+1 si N est le nombre d'implants.

De manière avantageuse, à chacune des longueurs différentes de tige est associée une texture spécifique présente sur les tiges ayant cette longueur et entre le repère visuel associé à cette longueur et le repère précédent. Cela facilite la perception par le chirurgien du repère à utiliser lors du forage d'un canal destiné à recevoir une tige donnée d'un implant choisi.

De manière préférée, les parties médianes ont une forme de boule, sphérique, ovale ou ellipsoïdale, avec des diamètres (ou dimensions transversales) qui augmentent avantageusement lorsqu'on passe d'un implant donné à un implant de taille supérieure.

Des objets, caractéristiques et avantages de l'invention ressortent de la description qui suit, donnée à titre d'exemple illustratif non limitatif, en regard du dessin annexé sur lequel :
- La figure 1 est une vue latérale d'un implant faisant partie d'un lot conforme à l'invention,
- La figure 2 est une vue latérale d'un foret unique faisant partie d'un tel lot conforme à l'invention, et
- La figure 3 est une vue latérale d'un lot de quatre implants et du foret associé.

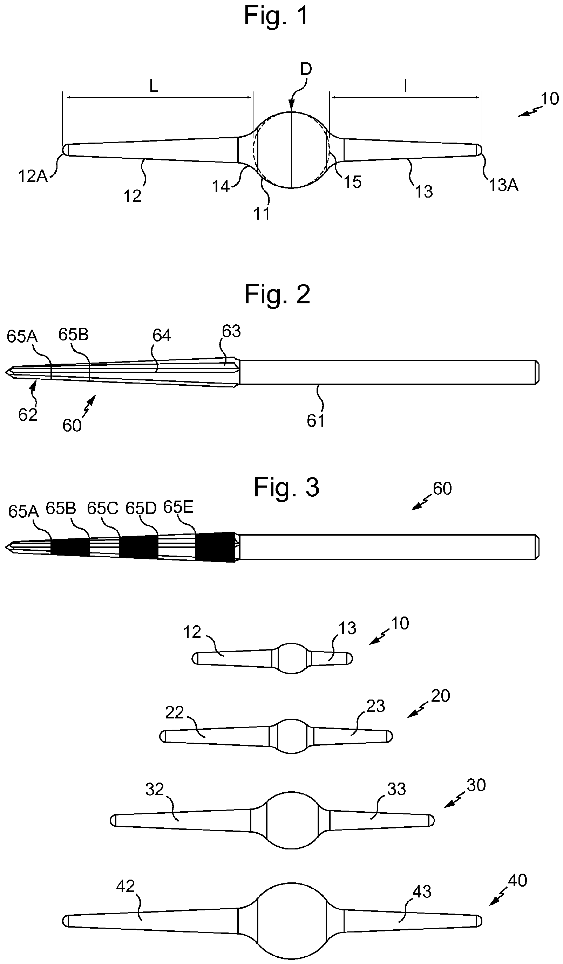

La figure 1 représente un implant 10 comportant une partie médiane 11 formée d'une boule d'un diamètre donné D et deux tiges 12 et 13 s'étendant l'une à l'opposé de l'autre à partir de cette partie médiane. Ces deux tiges ont des longueurs différentes, la tige 12, dite tige proximale (destinée à être implantée dans une phalange proximale) étant plus longue que la tige 13, dite tige distale (destinée à être implantée dans une phalange distale). Le rapport entre la longueur « I » de la tige distale et la longueur de la tige proximale « L » est par exemple compris entre 1/2 et 4/5. Ces longueurs sont mesurées entre l'extrémité 12A ou 13A (en pratique arrondie) des tiges et la partie médiane 11 ici assimilée à une sphère réelle (le contour fictif de cette sphère est représenté en tirets sur la figure 1).

Les tiges sont raccordées à la partie médiane par des zones de transition 14 et 15 ;

Selon l'invention, chacune des tiges a une forme conique avec une même conicité ; cette conicité est en pratique choisie entre 2 et 6°, par exemple 4.8°, valeur qui permet que le forage soit facilité par la conicité, sans toutefois impliquer une augmentation importante du diamètre d'entrée du canal ainsi formé.

Un tel implant est en pratique formé d'un matériau biocompatible, par exemple du silicone médical.

Le foret, désigné par la référence 60, comporte, au-delà d'une portion de maintien 61, une tige de travail 62 comportant des rainures longitudinales 63 (avec une éventuelle composante en hélice) bordant des arêtes de découpe 64 ; cette tige de travail a la même conicité que les tiges de l'implant 10. Sur cette tige de travail sont avantageusement visibles des repères 65A et 65B situés, par rapport à la pointe de la tige de travail, à des distances dont le foret doit pénétrer dans l'os pour permettre une bonne localisation d'une tige dans l'os de la phalange destinée à la recevoir (le repère 65A correspond ici à la profondeur de pénétration utile pour l'implantation de la tige distale et le repère 65B correspond à la profondeur de pénétration utile pour l'implantation de la tige proximale). Bien entendu, ces repères peuvent ne pas être présents, le chirurgien pouvant définir par lui-même la profondeur de forage à partir des dimensions de l'implant à mettre en place.

La figure 3 représente un exemple de lot d'implants comportant des implants conformes à celui de la figure 1 et un unique foret conforme à la figure 2. Les divers implants ont des tiges présentant une même conicité, égale à celle du foret. Le nombre d'implants au sein d'un lot est ici arbitrairement fixé à quatre.

Selon une particularité préférée des implants du lot, la tige proximale de l'implant le plus petit a la même longueur que la tige distale de l'implant de taille immédiatement supérieure. En d'autres termes, si l'on note 10, 20, 30 et 40 les quatre implants représentés sur la figure 3, la tige proximale 12 de l'implant a la même longueur que la tige distale 23 de l'implant 20, la tige proximale 22 de cet implant 20 a la même longueur que la tige distale 33 de l'implant 30, et la tige proximale 32 de cet implant 30 a la même longueur que la tige distale 43 de l'implant 40.

La figure 1 visant à visualiser la géométrie générale des implants 10 d'un lot conforme à l'invention, c'est par un choix arbitraire qu'il est indiqué qu'elle représente l'implant 10 de la figure 3 plutôt que, par exemple, l'implant 40.

On comprend qu'un avantage d'une telle gradation des tiges des implants est que, notamment, les moules de coulée d'un implant donné reprend, pour au moins l'une de ses tiges, la géométrie d'une partie du moule associé à un implant de taille immédiatement supérieure ou inférieure ; il en résulte une simplification dans la fabrication des moules.

Un autre avantage de cette gradation est que le foret peut comporter un nombre limité de repères visuels pour aider le chirurgien à identifier jusqu'à quel niveau de pénétration faire avancer le foret ; le premier repère 65A correspond à la profondeur de pénétration du foret pour forer le canal destiné à recevoir la tige distale de l'implant le plus petit, à savoir l'implant 10 ; mais le second repère 65B correspond à la profondeur de pénétration du foret pour forer le canal destiné à recevoir la tige proximale de cet implant 10, mais aussi le canal destiné à recevoir la tige distale de l'implant de taille immédiatement supérieure, à savoir l'implant 20 ; de même, le repère 65C correspond à la profondeur de pénétration associé à la tige proximale de l'implant 20 ainsi que de la tige distale de l'implant 30, et le repère 65D correspond à la profondeur de pénétration pour la tige proximale de l'implant 30 et de la tige distale de l'implant 40. Et le repère 65E correspond à la profondeur de pénétration du canal destiné à recevoir la partie proximale de l'implant le plus grand.

On peut apprécier qu'ainsi, il y a un nombre de repères à peine égal à N+1 si N est le nombre d'implants.

Pour faciliter encore plus l'ergonomie de l'ensemble, chaque tige peut avantageusement avoir une texture (il peut s'agir d'une couleur) spécifique, reproduite sur le foret. Dans un tel cas, de manière avantageuse, la texture de la tige distale 13 du plus petit implant 10 peut être reproduite sur le foret entre son extrémité et le premier repère 65A, la texture commune à la tige proximale 12 du premier implant et à la tige distale 23 de l'implant 20 peut être reproduite entre les premier et second repères 65A et 65B, la texture commune à la tige proximale 22 du second implant 20 et à la tige distale 33 du troisième implant 30 peut être reproduite entre les repères 65B et 65C, la texture commune à la tige proximale 32 du troisième implant et à la tige distale 43 du quatrième implant 40 peut être reproduite entre les repères 65C et 65D et la texture de la tige proximale 42 de ce dernier implant peut être reproduite entre les repères 65D et 65E. Le chirurgien peut ainsi savoir que, s'il veut implanter dans une phalange l'une des tiges de l'un des implants, il doit prolonger la pénétration du foret jusqu'à escamoter la portion du foret ayant la texture de la tige en question.

Une telle succession de textures est, de manière arbitraire, visualisée sur la figure 3 (sur le seul foret et non pas sur les implants) par une succession de zones claires et noires ; en variante ces textures peuvent être toutes différentes. On comprendra toutefois que, dans une version simplifiée, il peut être choisi de ne pas chercher à contrôler l'état de surface et/ou la couleur des tiges des divers implants.

De manière avantageuse, les longueurs des tiges présentent des écarts approximativement égaux ; ainsi à titre d'exemple, les longueurs présentent un écart de l'ordre de 4 à 6 mm, par exemple de 5 mm (avec par exemple une succession de longueurs du type 6, 11, 15, 20, 25 mm).

S'agissant des parties médianes, elles présentent de préférence une progression, choisie pour être compatible avec les variations de diamètre des tiges auprès de ces parties médianes (à titre d'exemple, les diamètres s'échelonnent comme suit : 5 - 5.5 - 8.5 - 11 mm).

En pratique un chirurgien, avant d'implanter effectivement un implant, fait des tests au moyen d'implants factices (en ce sens qu'ils sont représentatifs des vrais implants sans être ceux-ci), de manière à garantir que les tests ne risquent pas de dégrader l'implant qui sera finalement implanté ; ces implants d'essai, parfois appelés implants fantômes, présentent la même progression de longueur (en ce qui concerne les tiges) ou de diamètre (en ce qui concerne les parties médianes).

Dans une version simplifiée d'un lot conforme à l'invention, il peut n'y avoir d'identité entre la longueur d'une tige proximale d'un implant et celle de la tige distale d'un autre implant que pour certains seulement des implants, notamment lorsque la profondeur de pénétration de forage ne mérite d'être visualisée que pour certains des implants.

Par ailleurs, cette identité de longueur peut ne pas correspondre à des implants se suivant dans la succession des implants par taille ; ainsi, on peut prévoir que la tige proximale du plus petit implant ait la même longueur que la tige distale, non du deuxième implant, mais du troisième, que la tige proximale du second implant ait la même longueur que la tige distale du quatrième implant, et ainsi de suite, ce qui peut avoir comme avantage d'obtenir une plus grande souplesse dans le choix de l'implant à implanter.

## Revendications

1. Ensemble pour une arthroplastie du pied comportant une pluralité d'implants (10, 20, 30, 40) de tailles croissantes et d'un foret unique (60), chaque implant comportant à partir d'une portion médiane (11) une tige proximale (12, 22, 32, 42) et une tige distale (13, 23, 33, 43) plus courte que la tige proximale, **caractérisé en ce que** les tiges et le foret présentent des conicités égales comprises entre 2 et 6°.

2. Ensemble selon la revendication 1, dont la tige distale d'un implant a la même longueur que la tige proximale d'un autre implant.

3. Ensemble selon la revendication 1 ou la revendication 2, dont les tiges et le foret ont une conicité de 4.8° environ.

4. Ensemble selon l'une quelconque des revendications 1 à 3, dont le foret comporte sur sa tige de travail au moins un repère visuel (65A, 65B, 65C, 65D, 65E) définissant, à partir de la pointe de ce foret, une distance de pénétration égale à la longueur d'au moins une tige distale ou proximale d'un implant de ladite pluralité d'implants.

5. Ensemble selon l'une quelconque des revendications 1 à 3, dont les implants, classés par ordre de taille croissante, ont chacun (sauf le plus grand) une tige proximale dont la longueur est égale à celle de la tige distale de l'implant suivant.

6. Ensemble selon la revendication 5, dont le foret comporte sur sa tige de travail, pour chacune des longueurs différentes des tiges des implants de ladite pluralité d'implants, un repère visuel.

7. Ensemble selon la revendication 6, dans lequel à chacune des longueurs différentes de tige est associée une texture spécifique présente sur les tiges ayant cette longueur et entre le repère visuel associé à cette longueur et le repère précédent.

8. Ensemble selon l'une quelconque des revendications 1 à 7, dont les parties médianes ont une forme de boule.

9. Ensemble selon la revendication 8, dont les parties médianes ont des dimensions transversales qui augmentent lorsqu'on passe d'un implant donné à un implant de taille supérieure.

## Patentansprüche

1. Anordnung für eine Arthroplastik des Fußes, die eine Mehrzahl von Implantaten (10, 20, 30, 40) von steigender Größe und einen einzigen Bohrer (60) umfasst, wobei jedes Implantat von einem Mittelbereich (11) einen proximalen Schaft (12, 22, 32, 42) und einen distalen, kürzeren Schaft (13, 23, 33, 43) als der proximale Schaft aufweist, **dadurch gekennzeichnet, dass** die Schafte und der Bohrer eine Konizität zwischen 2 und 6° aufweisen.

2. Anordnung nach Anspruch 1, bei der der distale Schaft eines Implantats die gleiche Länge wie der proximale Schaft eines anderen Implantats aufweist.

3. Anordnung nach Anspruch 1 oder Anspruch 2, bei der die Schafte und der Bohrer eine Konizität von ungefähr 4,8° aufweisen.

4. Anordnung nach einem beliebigen der Ansprüche 1 bis 3, bei der der Bohrer an seinem Arbeitsschaft mindestens eine visuelle Markierung (65A, 65B, 65C, 65D, 65E) aufweist, die von der Spitze dieses Bohrers eine Eindringtiefe definiert, die gleich der Länge mindestens eines distalen oder proximalen Schafts eines Implantats der Mehrzahl von Implantaten ist.

5. Anordnung nach einem beliebigen der Ansprüche 1 bis 3, bei der die Implantate, klassifiziert nach einer Reihenfolge steigender Größe, jeweils (mit Ausnahme des größten) einen proximalen Schaft aufweisen, dessen Länge gleich der des distalen Schaftes des folgenden Implantats ist.

6. Anordnung nach Anspruch 5, bei der der Bohrer an seinem Arbeitsschaft für jede der unterschiedlichen Längen der Schafte der Implantate der Mehrzahl von Implantaten eine visuelle Markierung aufweist.

7. Anordnung nach Anspruch 6, bei der jeder der unterschiedlichen Schaftlängen eine spezifische, auf den Schäften vorhandene Textur zugeordnet ist, die diese Länge und zwischen der dieser Länge zugeordneten visuellen Markierung und der vorhergehenden Markierung aufweist.

8. Anordnung nach einem beliebigen der Ansprüche 1 bis 7, deren Mittelbereiche die Form einer Kugel aufweisen.

9. Anordnung nach Anspruch 8, deren Mittenbereiche Querabmessungen aufweisen, die ansteigen, wenn von einem gegebenen Implantat auf ein Implantat größerer Abmessung übergegangen wird.

## Claims

1. A kit for foot arthroplasty comprising a plurality of implants (10, 20, 30, 40) of increasing sizes and a single bit (60), each implant comprising, starting from a median portion (11), a proximal stem (12, 22, 32, 42) and a distal stem (13, 23, 33, 43) shorter than the proximal stem, **characterized in that** the stems and the bit have equal conical tapers comprised between 2 and 6°.

2. A kit according to claim 1, wherein the distal stem of an implant has the same length as the proximal stem of another implant.

3. A kit according to claim 1 or claim 2, wherein the stems and the bit have a conical taper of 4.8° approximately.

4. A kit according to any one of claims 1 to 3, wherein the bit comprises on its working stem at least one visual marker (65A, 65B, 65C, 65D, 65E) defining, from the point of that bit, a penetration distance equal to the length of at least one distal or proximal stem of an implant of said plurality of implants.

5. A kit according to any one of claims 1 to 3, wherein the implants, classified in increasing order of size, each have (except for the largest) a proximal stem of which the length is equal to that of the distal stem of the next implant.

6. A kit according to claim 5, wherein the bit comprises a visual marker on its working stem for each of the different lengths of the stems of the implants of said plurality of implants.

7. A kit according to claim 6, wherein with each of the different lengths of stem there is associated a specific texture present on the stems having that length and between the visual marker associated with that length and the preceding marker.

8. A kit according to any one of claims 1 to 7, wherein the median parts are ball-shaped.

9. A kit according to claim 8, wherein the median parts have transverse dimensions which increase on going from a given implant to an implant of larger size.
